# EUROPEAN PATENT APPLICATION

(11) **EP 4 043 477 A1**
(43) Date of publication of application: **17.08.2022**
(21) Application number: 22151823.6
(22) Date of filing: 17.01.2022
(51) Int. Cl.: C07J 31/00

(54) **PROCESS FOR THE PREPARATION OF FULVESTRANT**

(30) Priority: 12.02.2021 IT 202100003176
(71) Applicant: Farmabios S.p.A., 27027 Gropello Cairoli (Pavia) (IT)
(72) Inventor: GABOARDI, Mauro, 28100 Novara (NO) (IT); AROSIO, Roberto, 23862 Civate (LC) (IT); ZERILLI, Francesco, 27100 Pavia (PV) (IT)
(74) Representative: Longoni, Alessandra

(57) **Abstract**

The present invention relates to a process for the preparation of Fulvestrant, comprising the recovery of by-products deriving from the oxidation of the corresponding sulfide, which are subjected to a reduction reaction in the presence of specific reducing agents

## Description

The present invention relates to a process for the preparation of Fulvestrant, comprising the recovery of the by-products deriving from the oxidation of the corresponding sulfide, which are subjected to a reduction reaction in the presence of specific reducing agents.

Fulvestrant is an antiestrogen used in postmenopausal women with locally advanced or metastatic estrogen receptor positive breast cancer. The active ingredient Fulvestrant, also known as (7α, 17β)-7-[9-[(4,4,5,5,5-pentafluoropentyl) sulfinyl]nonyl]estra-1,3,5(10)-triene-3,17-diol, has the following formula (I) 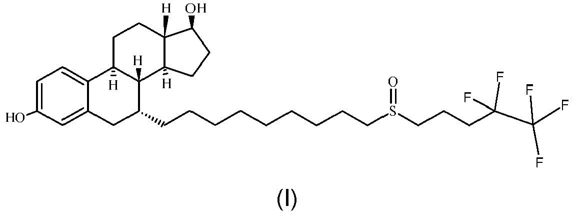 and is marketed as a medicinal product in the form of a solution for intramuscular injection under the brand name Faslodex^{®}.

Fulvestrant present in the pharmaceutical form is a mixture of two epimeric diastereomers on the sulfur atom. These two diastereomers are identified as Fulvestrant sulfoxide A and Fulvestrant sulfoxide B.

For use in therapy, the regulatory authorities require to control the amount of each diastereomer in the finished product, keeping the ratio between isomer A and isomer B between 42-48/58-52.

Several processes are known for the preparation of Fulvestrant.

For example, US 4,659,516 discloses a process for the preparation of Fulvestrant according to Scheme 1 reported below:

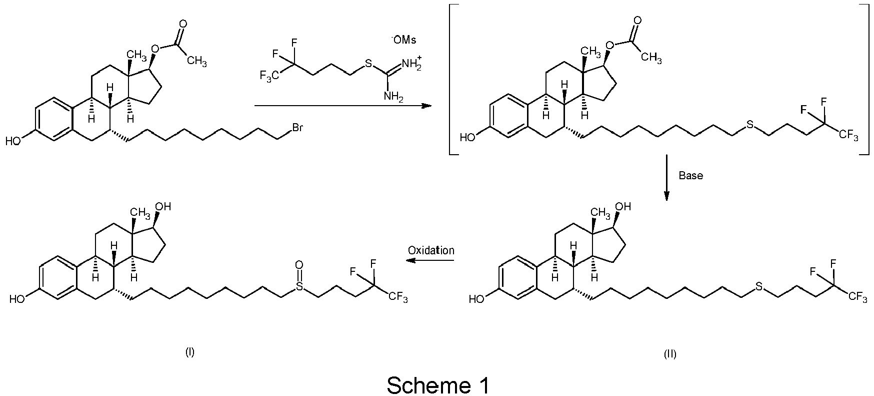

The crude Fulvestrant (I) obtained by oxidation of the corresponding sulfide (II), is purified with known techniques such as chromatography on silica and crystallization from ethyl acetate to isolate Fulvestrant having an isomeric ratio A/B between 42-48/58-52.

However, in addition to obtaining the desired Fulvestrant, the above process presents the problem of the formation of significant quantities of some by-products deriving from the oxidation reaction, such as the corresponding sulfone and Fulvestrant having an isomeric ratio A/B outside the permitted limit that comes from the chemical synthesis obtained in a 50/50 ratio with respect to the Fulvestrant of interest. This implies that after purification, the product falls within the diastereosomeric ratio specifications reported above, while the recovery fractions, isolated after said purification of the crude Fulvestrant contain large quantities of the aforementioned diastereoisomers in a ratio of about 60/40, not suitable for commercial purposes; these fractions are normally discarded thus lowering the overall yield of the process. CN 106146599 proposes a solution to the problem, i.e. a process for the preparation of Fulvestrant comprising the recycling of the recovery fractions which are subjected to a reduction reaction in the presence of strong reducing agents to obtain the corresponding sulfide and subsequent oxidation to form the desired Fulvestrant. In particular, in this document, the reduction reaction is carried out by various methodologies, such as catalytic hydrogenation, acid-catalyzed metal reaction, borohydride in the presence of a Lewis acid, lithium aluminum hydride or diisobutyl aluminum hydride, sodium borohydride-iodine or boranes, or a combination of dichlorosulfoxide-triphenyl phosphine or oxalyl chloride-triphenyl phosphine.

Similarly, the patent CN 107698647 describes a preparation method of Fulvestrant comprising a reduction reaction of the recycled recovery fractions with reducing agents such as sodium bromide or sodium iodide in the presence of an acid such as HBr, HI, BF₃·Et₂O or pTSA. The sulfide thus obtained is then oxidized to lead to the desired Fulvestrant.

However, the prior art makes use of very strong reducing reagents or strongly acidic conditions which could lead to the degradation of the Fulvestrant molecule and therefore to the formation of numerous impurities.

Therefore, there is a need for an effective and safe process of preparation of Fulvestrant that does not use strong reducing reagents.

We have now found a process for the preparation of Fulvestrant which does not have the drawbacks of the known methods and which allows to recover, by recycling the oxidation by-products, an amount of Fulvestrant of about 50-70% referred to the quantity of eliminated Fulvestrant.

An object of the present invention is therefore a process for the preparation of Fulvestrant comprising a recycling step of the reaction by-products formed in the oxidation reaction from sulfide (II) to Fulvestrant and reduction of the same to restore the sulfide (II),
characterized in that said reduction reaction takes place in the presence of a reducing agent selected from sodium bisulfite, sodium metabisulfite, diiron-nonacarbonyl phenylsilane.

The process of the present invention is represented in Scheme 2 below.

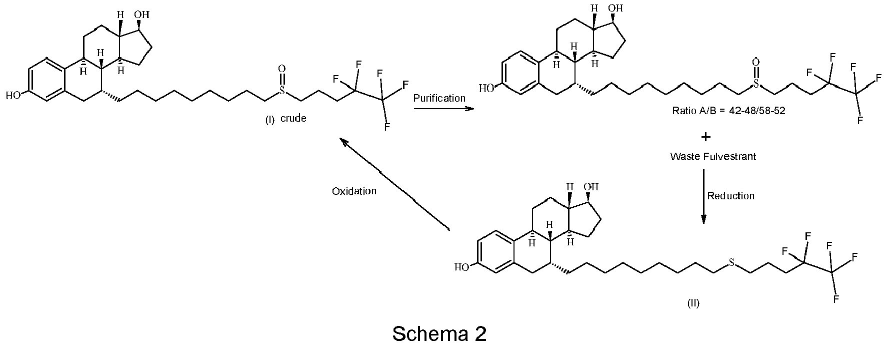

According to the present invention, crude Fulvestrant (I) is obtained according to methods described in the art, such as for example in US 4,659,516.

The purification of the crude is carried out using techniques well known to those skilled in the art, such as silica chromatography and subsequent crystallization in a suitable solvent.

In particular, the first purification by chromatography allows to separate the Fulvestrant from the sulfone impurity present in significant quantities in the head fractions of the column.

The subsequent fractions containing the desired Fulvestrant are subjected to crystallization, preferably in ethyl acetate, to eliminate the Fulvestrant having an unsuitable A/B isomeric ratio, i.e. about 60/40.

These two purification steps lead to obtaining the Fulvestrant of interest with about 70% of the overall yield of the process.

Therefore, in order to recover greater quantities of product, the waste fractions constituted by the head fractions of the column enriched in sulfone and the crystallization mother liquors containing Fulvestrant having an isomeric ratio A/B that does not comply with the release specifications (typically A/B 60/40) are combined and brought to residue.

According to the present invention, such fractions identified as waste Fulvestrant are subjected to a reduction reaction so as to restore the sulfide (II).

Typically, the waste Fulvestrant contains about 90% of Fulvestrant having an isomeric ratio A/B not suitable for commercial purposes, i.e. about 60/40, as well as about 10% of sulfone (III) of formula:

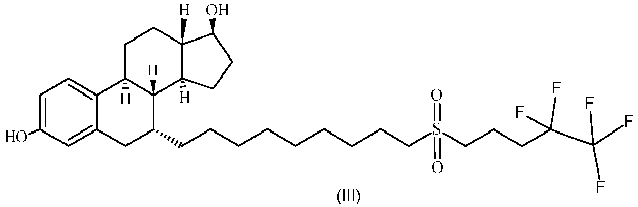

Preferably, the waste Fulvestrant is dissolved in an organic solvent such as for example toluene, tetrahydrofuran, chloroform, acetonitrile, methyl tetrahydrofuran, dioxane. Dissolution is conveniently carried out at room temperature.

A reducing agent selected from sodium bisulfite, sodium metabisulfite, diiron-nonacarbonyl phenylsilane is added to this solution.

According to a preferred embodiment, when a reducing agent selected from sodium bisulfite and sodium metabisulfite is used, a catalytic amount of iodine is added to the reaction mixture.

Said reducing agent is preferably introduced into the reaction mixture in a molar quantity between 1.0 and 2.0 equivalents with respect to the molar quantity of waste Fulvestrant, more preferably comprised between 1.0 and 1.5 equivalents.

Furthermore, according to the present invention, this reducing agent can be added to the reaction mixture as a solid or as an aqueous solution.

Preferably, the reducing agent is used as an aqueous solution as it is commercially available.

According to the invention, said aqueous solution has a concentration between 10 and 45%, preferably 40%.

After the addition of the reducing agent, the reaction mixture is heated to reflux of the solvent and left under stirring, generally between 2 and 18 hours, until total conversion of the sulfoxide to sulfide (II) is obtained.

The reducing agents identified by the Applicant are mild agents and particularly suitable for the reduction of waste Fulvestrant for the preparation of sulfide (II). These agents in fact allow to selectively convert the Fulvestrant having an unsuitable A/B isomeric ratio into the sulfide (II), without degrading or reducing the sulfone (III) present in the waste Fulvestrant.

Furthermore, unlike other mild reducing agents tested by the Applicant, such as sodium sulfite, for example, the reducing agents used in the present invention bring the reduction reaction to completion. A reaction mixture is then obtained which can possibly be subsequently purified to isolate the sulfide (II).

In fact, as it is well known to the skilled person, the mixture can be subjected to various purification operations in order to separate the sulfide (II) from the sulfone (III), such as silica chromatography.

Preferably, said mixture is purified by means of a chromatographic column to isolate the sulfide (II) free from the sulphone (III).

According to a preferred embodiment, the sulfide (II) obtained with the process of the present invention is subsequently subjected to an oxidation reaction to prepare Fulvestrant (I).

The experimental conditions for carrying out this reaction are those known to the skilled person.

In particular, the residue containing the sulfide (II) is dissolved in at least one organic solvent, such as for example tetrahydrofuran, methanol, and mixtures thereof as described in the cited prior art.

Preferably, said residue is dissolved in a mixture of tetrahydrofuran and methanol. The solution thus obtained is cooled to a temperature of about 5°C and an oxidizing agent is added.

Examples of oxidizing agents according to the present invention are sodium (meta) periodate, meta-chloroperbenzoic acid.

Generally after 15-18 hours, the reaction is complete and is brought to residue.

The residue thus obtained is then purified by techniques known in the art, such as silica chromatography and crystallization from ethyl acetate to provide the desired Fulvestrant.

As it is clear to the expert in the field, it is therefore possible to isolate again the recovery fractions resulting from the purification of the raw reaction that still contain Fulvestrant not suitable for commercial purposes in addition to sulfone (III).

The recovery of these fractions to be subjected to reduction and subsequent oxidation can be carried out one or more times depending on the quantities to be purified.

Thanks to the process of the present invention and in particular to the use of specific reducing agents, the Applicant has managed to recover 50-70% of the Fulvestrant which was previously lost in the final crystallization and in the mixed chromatographic fractions with a significantly higher yield than the process described in US 4,659,516 which does not include the recycling step.

A further object of the present invention is the Fulvestrant obtained by the process of the present invention.

The present invention will now be illustrated by means of some examples, which should not be seen as limiting the scope of the invention.

### Experimental part

### Example 1: Procedure for obtaining waste Fulvestrant

The fractions of the chromatographic column containing Fulvestrant with the diastereoisomeric ratio not complying with the specifications and enriched with sulfone and the mother liquors of final crystallization are combined. The solvent is distilled under vacuum to a residue. The residue is added with toluene and the mixture is heated up to 70°C. It is cooled to 20°C and the crystallization of the product is obtained. The product is filtered on Buchner and washed with toluene. The product is dried in an oven at 50°C under vacuum for 15 h.

### EXAMPLES OF REDUCTION FROM SULFOXIDE TO SULFIDE

### Example 2

Waste Fulvestrant (5 g, 8.24 mmol) is loaded into a 3-necked flask equipped with a mechanical stirrer, thermometer and condenser. Chloroform (25 ml) is charged under nitrogen and there is complete dissolution. Sodium metabisulfite (1.67 g, 8.8 mmol) and iodine (0.20 g, 0.8 mmol) are added to the solution. The reaction mixture is heated under reflux and monitored overtime. After 18 h the reaction is complete (conversion> 99%). The reaction mixture is cooled to 25 ± 5°C and demineralized water (15 ml) is added. The phases are separated and the organic phase is washed once with a saturated sodium chloride solution (5 ml). The phases are separated and the organic phase is concentrated under vacuum to a residue. About 4.5 g of product are obtained as thick oil.

### Example 3

Waste Fulvestrant (20 g, 32.97 mmol) is loaded into a 3-necked flask equipped with a mechanical stirrer, thermometer and condenser. THF (100 ml) is charged under nitrogen and there is complete dissolution. Sodium metabisulfite (6.9 g, 36.26 mmol) and iodine (0.83 g, 3.3 mmol) are added to the solution. The reaction mixture is heated under reflux and monitored over time. After 8 h the reaction is complete (conversion> 99%). The reaction mixture is cooled to 25 ± 5°C and demineralized water (60 ml) and toluene (100 ml) are added. The phases are separated and the organic phase is washed once with a saturated sodium chloride solution (5 ml). The phases are separated and the organic phase is dehydrated with magnesium sulphate and concentrated under vacuum to a residue. About 20.3 g of product are obtained as thick oil.

### Example 4

Waste Fulvestrant (10 g, 16.5 mmol) is loaded into a 3-necked flask equipped with a mechanical stirrer, thermometer and condenser. THF (50 ml) is charged under nitrogen and there is complete dissolution. Sodium bisulfite (1.89 g, 18 mmol) and iodine (0.42 g, 1.65 mmol) are added to the solution. The reaction mixture is heated under reflux and monitored over time. After 3 hours and 30 minutes the reaction is complete (conversion> 99%). The reaction mixture is cooled to 25 ± 5°C and demineralized water (60 ml) and toluene (100 ml) are added. The phases are separated and the organic phase is washed once with a saturated sodium chloride solution (5 ml). The phases are separated and the organic phase is dehydrated with magnesium sulphate and concentrated under vacuum to a residue. About 4.8 g of product are obtained as thick oil.

### Example 5

Waste Fulvestrant (5 g, 8.24 mmol) is loaded into a 3-necked flask equipped with a mechanical stirrer, thermometer and condenser. Toluene (25 ml) is charged under nitrogen and there is complete dissolution. Diironnonacarbonyl (0.3 g, 0.82 mmol) and phenyl silane (1 ml, 8.24 mmol) are added to the solution. The reaction mixture is heated to 100°C and monitored over time. After 3 hours and 30 minutes the reaction is complete. The reaction mixture is cooled to 25 ± 5°C and the solution is filtered on a panel of tonsil earth. It is washed with toluene and evaporated under vacuum to a residue. About 6 g of product are obtained as thick oil (containing phenylsilane).

### Example 6

Waste Fulvestrant (50 g, 82.4 mmol) is loaded into a 3-necked flask equipped with a mechanical stirrer, thermometer and condenser. THF (250 ml) is charged under nitrogen and there is complete dissolution. 40% sodium bisulfite (30 g, 115 mmol) and iodine (2.08 g, 8.2 mmol) are added to the solution. The reaction mixture is heated under reflux and monitored over time. After 2 h the reaction is complete (conversion> 99%). The reaction mixture is cooled to 25 ± 5°C and demineralized water (60 ml) and toluene (100 ml) are added. The phases are separated and the organic phase is washed once with a saturated sodium chloride solution (5 ml). The phases are separated and the organic phase is dehydrated with magnesium sulphate and concentrated under vacuum to a residue. About 52 g of product are obtained as thick oil.

### Example 7 (Comparative)

Waste Fulvestrant (5 g, 8.24 mmol) is loaded into a 3-necked flask equipped with a mechanical stirrer, thermometer and condenser. THF (25 ml) is charged under nitrogen and there is complete dissolution. Sodium sulfite (1.14 g, 8.8 mmol) and iodine (0.21 g, 0.82 mmol) are added to the solution. The reaction mixture is heated under reflux and monitored over time. The reaction is slow, after 23 h the conversion is 66%. The reaction is unsatisfactory and is not processed.

### EXAMPLES OF OXIDATION FROM SULFIDE TO SULFOXIDE

### Example 8

Sulfide (II) (20 g, 33.8 mmol obtained from example 3) is charged into a 500 ml 3-necked flask, equipped with a mechanical stirrer, thermometer, and under nitrogen atmosphere, and dissolved in THF (235 ml) and methanol (60 ml). It is cooled to 5°C and a solution of sodium (meta) periodate (11.3 g, 49 mmol) in water (80 ml) is added. It is left to react at room temperature for 15-18h. At the end of the reaction, the organic solvents are removed by distilling under vacuum and methylene chloride (200 ml) is added. The phases are separated. It is washed with water, the solvent is removed under vacuum and crystallized from toluene to obtain crude Fulvestrant (13 g). Crude Fulvestrant is then purified by silica chromatography (300 g, eluent: methylene chloride / methanol 98: 2) and the cleaned fractions are crystallized from ethyl acetate. 9.4 g of product are obtained in compliance with the specifications according to the pharmacopoeias.

## Claims

1. Process for the preparation of Fulvestrant comprising a recycling step of the reaction by-products formed in the oxidation reaction from sulfide (II) to Fulvestrant and reduction thereof to restore the sulfide (II), 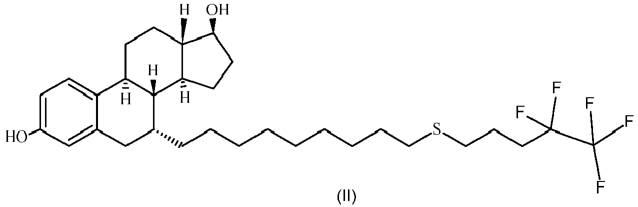 **characterized in that** said reduction reaction is performed in presence of a reducing agent selected from sodium bisulfite, sodium metabisulfite, diiron-nonacarbonyl phenylsilane.

2. Process according to claim 1, **characterized in that** when said reducing agent is selected from sodium bisulfite and sodium metabisulfite, a catalytic amount of iodine is added.

3. Process according to any one of the preceding claims, **characterized in that** said reducing agent is used as solid or as an aqueous solution, preferably as an aqueous solution.

4. Process according to claim 3, **characterized in that** said aqueous solution has a concentration between 10% and 45%, preferably of about 40%.

5. Process according to any one of the preceding claims, **characterized in that** said reducing agent is used in a molar amount comprised between 1.0 and 2.0 equivalents with respect to the molar amount of the by-products, preferably between 1.0 and 1.5 equivalents.

6. Process according to any one of the preceding claims, **characterized by** further comprising an oxidation reaction to convert the sulfide (II) into Fulvestrant.

7. Process according to claim 6, **characterized in that** said oxidation reaction is performed in the presence of an oxidizing reagent selected from sodium (meta)periodate and meta chloroperbenzoic acid.

8. Fulvestrant obtained by the process according to any one of claims 1-7.
